# EUROPEAN PATENT APPLICATION

(11) **EP 2 248 551 A1**
(43) Date of publication of application: **10.11.2010**
(21) Application number: 09159371.5
(22) Date of filing: 05.05.2009
(51) Int. Cl.: A61N 5/10

(54) **Method for the verification of a radiotherapy treatment apparatus**

(71) Applicant: 7Sigma N.V., 3150 Tildonk (BE)
(72) Inventor: Van Esch, Ann, 3150, Tildonk (BE); Huyskens, Dominique, 3150, Tildonk (BE)
(74) Representative: Callewaert, Koen

(57) **Abstract**

The invention concerns a method for the verification of a radiotherapy treatment apparatus and for the verification of treatment preparation software and control software and a corresponding apparatus. The apparatus has at least one radiation source mounted to a gantry for directing one or multiple radiation beams to a target from one or from multiple angles of beam incidence, wherein the beams can be altered or modulated as a function of gantry angle, position or time. In this method a treatment plan is obtained for treatment of a target in a patient or in a phantom or for apparatus performance control, said treatment plan comprising defining said radiation beams to be directed from one or from multiple angles of incidence.

## Description

### Technical field of the invention:

The invention concerns a method for the verification of a radiotherapy treatment apparatus and for the verification of treatment preparation software and control software. The apparatus has at least one radiation source mounted to a gantry for directing one or multiple radiation beams to a target from one or from multiple angles of beam incidence, wherein the beams can be altered or modulated as a function of gantry angle, position or time. In this method a treatment plan is obtained for treatment of a target in a patient or in a phantom or for apparatus performance control, said treatment plan comprising defining said radiation beams to be directed from one or from multiple angles of incidence.

### State of the art of radiotherapy treatments:

Radiotherapy cancer treatments make generally use of megavoltage photon beams that are directed to a target formed around a tumour that is to be treated. The goal of radiotherapy is to deliver a lethal dose to the tumour volume, while sparing the healthy tissue as much as possible.

Treatments were traditionally made up of a few simple open (homogeneous) or wedged fields, targeting the tumour from different angles of incidence by means of radiation beams. Modem day linear accelerators are equipped with a multileaf collimator (MLC), allowing easy delivery of conformal homogeneous (or wedged) treatment fields.

A big step forward was the introduction of IMRT (Intensity Modulated Radiation Therapy): computer generated movements of the multileaf collimator result in an intensity modulated two-dimensional machine output for every treatment beam angle. IMRT treatments most commonly deliver intensity modulated 2D photon fluences from a beforehand selected number of beam incidences. There exists an increasing interest in the use of arc or rotational rather than fixed gantry treatment delivery, allowing the use of a 360 degree beam incidence: The photon beam then rotates around the patient while delivering the requested dose segment to the target as a function of beam angle. The complexity of such an arc treatment can vary from very simple conformal arc delivery to highly complex inverse planned MLC movements.

### Quality assurance of radiotherapy treatments:

At the start of a radiotherapy treatment, the patient is set up on a treatment couch as he or she will be during the actual treatment. Reference X-ray images are then acquired, these can be 2D projected radiographs or 3D CT images. Modem, conformal radiotherapy is mostly based on a volumetric CT image, on which the target structure(s) and organs at risk have been outlined by qualified medical staff, mostly by the radiation oncologist.

Based on these contoured structures and the patient CT, the radiotherapy treatment planner uses treatment planning system (TPS) software to calculate dose distributions in the patient and to propose an optimal treatment solution. In order to assure correct delivery of the radiation beams to the patient, multiple aspects need to be verified. These include the accuracy of the dose calculation, the accuracy of the treatment delivery (i.e. the performance of the treatment unit) and the precision with which the patient is set up. Monitoring the accuracy of the dose calculation and treatment delivery is the responsibility of the medical physicist. Numerous tools are available to verify the patient positioning. Most modem today linear accelerators have a portal imager mounted on the treatment unit to allow imaging of the shape of each treatment field and the projection of the patient's anatomy within each treatment field. The portal imager is fit to measure megavoltage (MV) photon beams.

Single point dose measurements in the centre of homogeneous open or wedged beams at the start of every patient treatment session are often regarded as sufficient to assess the correct dose delivery of the treatment plan. However, alongside the implementation of IMRT came the need for 2D verification of the intensity modulated dose delivery. Two approaches were adopted.

The most straightforward one is to deliver the patient's treatment beam to a test setup in which the dose is then recalculated and measured by means of a sensitive film or a 2D array detector. This is a very time and resource consuming verification procedure.

A much more efficient procedure to verify the 2D photon fluence is by means of the portal imager device that is often mounted on the linear accelerator for patient imaging purposes. This verification procedure is referred to as 'portal dosimetry'. Based on the expected 2D photon fluence output and the response characteristics of the portal imager, a 2D portal dose image is predicted and measured for every treatment beam. Measurement and prediction are compared to assess the quality of the treatment delivery. This verification is mostly performed without a patient in the beam. Some research groups have worked on the use of portal dose images during the actual patient treatment: some have tried to predict the expected portal dose image, taking the patient anatomy into account while some have tried to use the measured portal dose image to reconstruct the actual dose delivery in the patient.

Although numerous publications exist on the subject, until now, the only procedure that has been made commercially available and that has become widespread in daily routine is the portal dosimetry without a patient in the beam.

Arc treatments can be among the most complex dose delivery methods and require careful verification. The first verification approach as developed for static gantry IMRT verification is also applicable to arc treatments: the whole treatment is transferred to a phantom on which the dose is recalculated, measured and compared. The portal dosimetry verification procedure is not as trivial to transfer. Some simplified work on a 2D portal dose verification for a selection of gantry angles has already been done, but adds nothing fundamentally new to the existing procedures.

The existing methods for radiation beam verification are mostly based on acquired dose data in a medium and are therefore inherently medium-dependent. In particular, in existing methods, the acquired dose that is defined as the absorbed energy per unit mass is measured in a medium, especially in a tissue-equivalent medium such as water or water-equivalent compounds.

Although a number of 3D verification methods currently exist, these are actually based on comparing a 3D dose calculation with a limited number of 2D dose measurements. These measurements are performed by means of a sensitive film, ion chamber or diode arrays.

When aiming for 3D rather than 2D (or 1D) verification of a treatment plan, the only currently available full 3D measurement method is the dose-based gel-dosimetry. In this existing method a container of an appropriate size and shape is filled with a special gel of which the characteristics change as a function of absorbed dose. After irradiation, the gel container needs to be scanned in an MRI and the scan needs to be converted to an absorbed dose matrix by means of a calibration procedure. This is not a solution fit for clinical routine as the gel itself is very expensive and difficult to handle. In addition, availability of an MRI for data read-out is in most hospitals not feasible because of their extremely high patient load.

Further, radiotherapy linear accelerators require regular monitoring of the machine performance, focussing on both the mechanical and dosimetric stability of the treatment unit, such as the main collimator calibration, the MLC calibration and motor wear, the gantry and collimator rotational accuracy, the stability of the machine output intensity and energy spectrum, the flatness and symmetry of the treatment beam, etc...

### Object and summary of the invention:

The invention, inter alia, aims to provide a three dimensional verification method that is medium independent and that is optimized for efficiency in clinical routine. Thus the invention aims to eliminate the influence from the dose absorbing medium, allowing a more direct validation of the actual versus expected machine performance.

To this aim, in the method, according to the invention, it is verified whether the three dimensional photon density matrix, or thus the photon 'cloud', as delivered by the treatment apparatus is in agreement with the theoretically expected photon cloud.

According to a preferred embodiment of the method, according to the invention, this method comprises the steps of
- obtaining a treatment plan for treatment of a target in a patient or in a phantom or for apparatus performance control, said treatment plan comprising defining said radiation beams to be directed from one or from multiple angles of incidence;
- predicting a three-dimensional photon distribution in air on basis of said treatment plan;
- generating said radiation beams in air, wherein the radiation beams are defined by the treatment plan, while measuring radiation from said radiation beams by means of a detector device and calculating a corresponding three-dimensional in-air photon distribution taking into account the measured radiation and its angular distribution;
- comparing and evaluating the three-dimensional in-air photon distribution predicted on basis of said treatment plan and the calculated three-dimensional in-air photon distribution based on the measured radiation.

In an advantageous embodiment of the method, according to the invention, radiation from said radiation beams is measured as a function of gantry angle when rotating the gantry around a centre or an axis of rotation.

In a particular interesting embodiment of the method, according to the invention, said three-dimensional photon distribution is predicted on basis of said treatment plan by superposing individual three-dimensional photon distributions of said radiation beams calculated for each gantry angle or by integrating the three dimensional photon distribution of said radiation beams over all angles.

In an advantageous manner, said three-dimensional photon distribution is predicted on basis of said treatment plan by calculating the photon flux in a plane perpendicular to the central axis of said radiation beams, wherein this plane preferably comprises the isocentre through which the central axis of the radiation beams passes for different gantry angles, and subsequently transforming this photon flux into said three-dimensional photon distribution.

In a particularly advantageous manner said photon flux is calculated taking into account mechanical parameters of the radiotherapy treatment apparatus such as absorption characteristics of mechanical parts of the apparatus, wherein these characteristics are expressed in terms of changes in photon intensity and/or photon energy spectrum on the central axis of the radiation beam as well as off this axis.

Preferably, measuring radiation from said radiation beams is performed by providing a portal imager defining a plane of measurement, preferably mounted to said gantry, whereby portal dose images generated by the portal imager are determined as a function of the gantry angle.

Advantageously, measuring radiation from said radiation beams comprises acquiring individual images of the radiation beams in a plane of measurement perpendicular to the central axis of this beam, whereby the images are stored together with the corresponding gantry angle.

In a preferred embodiment of the method, a set of transformation matrices is determined for converting said images into a photon flux, these transformation matrices comprising at least one of the following:
- a correction matrix for background subtraction obtained by acquiring an image without generating a radiation beam;
- a relative sensitivity correction matrix for correcting differences in sensitivity between individual pixels of said detector device;
- a matrix for the correction of scatter effects of the detector device construction, wherein scatter and backscatter effects are quantified by means of a series of image acquisitions for different field sizes of the radiation beam and different positions of the detector device;
- a correction matrix for the spectral dependence of the detector device

The invention also pertains to a radiotherapy treatment apparatus permitting comparing and evaluating a three-dimensional photon distribution predicted on basis of a treatment plan and a calculated three-dimensional photon distribution based on a measured radiation of a radiation beam. This apparatus comprises a radiation source for generating a radiation beam, wherein the radiation source is mounted to a gantry that can be rotated around a centre or axis of rotation, and a portal imaging device that is capable of generating a portal image in response to said radiation beams. Further, the apparatus is provided with means for acquiring a gantry angle associated with a corresponding portal image and for storing this gantry angle alongside the corresponding portal image.

### Brief description of the drawings

Other particularities and advantages of the invention will become clear from the following description of a few specific embodiments of the method and the apparatus according to the invention; this description is given as an example only and does not restrict the scope of the claimed protection in any way; the reference figures used hereafter refer to the accompanying drawings.
Figure 1 is a schematic drawing of a radiotherapy treatment apparatus for application of the method according to the invention.
Figure 2 is a schematic drawing of a photon distribution predicted on basis of a treatment plan.
Figure 3 shows a schematic flow sheet with some method steps for converting a raw image generated by a portal imager into a photon flux.

In the different drawings, the same reference figures refer to identical or analogous elements.

### Detailed description of the invention

In general, this invention concerns a methodology and an apparatus adapted to the three dimensional verification of arc treatments. The method aims to verify if the three dimensional photon density matrix (or photon 'cloud') as delivered by a treatment machine, in particular a radiotherapy apparatus, is in agreement with the theoretically expected photon cloud.

The method according to the invention is intended to be used with a radiotherapy apparatus that can generate megavoltage photon beams

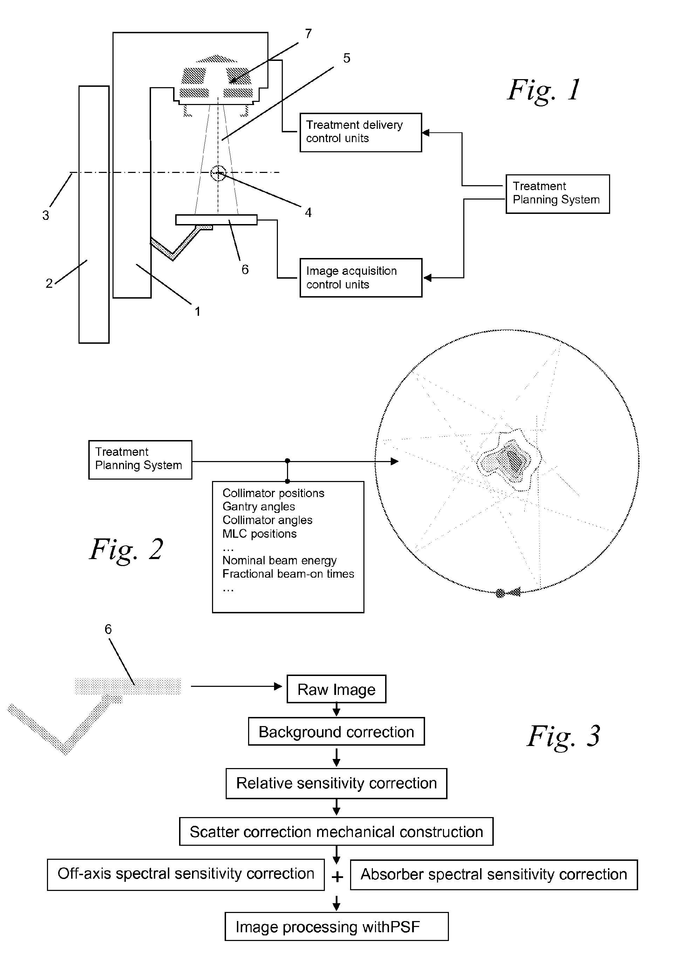

In figure 1 an apparatus for applying the method, according the invention is represented. This apparatus comprises a gantry 1 that is mounted to a support structure 2 such that the gantry 1 can rotate around an axis of rotation 3 or around a centre point 4. This rotation of the gantry 1 defines the gantry angle and thus the angle of beam incidence. The gantry 1 comprises a radiation source for generating a radiation beam 5. Further, a portal imaging device 6, also named portal imager, is mounted to the gantry 1 such that the radiation beam 5 impinges upon this device 6 in order to generate an image of the radiation beam 5. The portable imaging device 6 thus is moving together with the gantry 1. Further, the gantry 1 has a treatment head 7 that has control units for modulating or altering the radiation beam 5 and is generally provided with, amongst others, a linear accelerator equipped with a multileaf collimator (MLC).

For treatment of, for example, a tumour in a patient, a treatment plan is obtained or generated by means of so-called treatment planning software. This software determines, amongst others, the radiation beams that are required for treatment of a target, i.e. a tumour, and the angle of incidence of these beams taking into account the parameters of the radiotherapy apparatus. This angle of incidence is associated with the gantry angle.

Before applying the treatment plan to a patient, it has to be verified whether the application of the treatment plan by the radiotherapy apparatus is in agreement with the theoretically expected treatment.

In the method according to the invention, this is done by predicting a theoretical three dimensional photon distribution in air and by generating and measuring radiation beams in air by means of the treatment apparatus, wherein the radiation beams are defined by the treatment plan.

The radiation from said radiation beams is measured by means of the portal imaging device and, subsequently, a corresponding three-dimensional in-air photon distribution is calculated taking into account the measured radiation and its angular distribution.

The three-dimensional in-air photon distribution predicted on basis of said treatment plan and the calculated three-dimensional in-air photon distribution based on the measured radiation is then compared and evaluated in order to check whether the generated radiation is in agreement with the treatment plan.

For predicting the three dimensional photon distribution, also called three dimensional photon cloud, in air, input obtained from the treatment planning software is used. This input comprises both mechanical information and dosimetric information. The mechanical information relates, for example to the gantry angle, the collimator angle, the collimator coordinates, the MLC position, etc., whereas the dosimetric information concerns features such as, among others, beam-on time, treatment energy, treatment beam spectrum and treatment beam profile, etc.

Using input obtained from the treatment planning software, the theoretically expected two dimensional photon fluence is calculated as a function of gantry angle and superposed to generate a three dimensional photon density matrix in air. The three dimensional photon-cloud in air is, in particular, predicted by calculating and superposing the theoretical treatment apparatus output from all gantry angles.

The prediction of the theoretical output of the treatment apparatus is schematically represented in figure 2.

The total three dimensional photon-cloud is, in particular, calculated as a superposition of the individual three dimensional contributions from all gantry angles. For every gantry angle, the contribution to the total photon-cloud is calculated by first calculating the photon flux in a plane containing the isocentre, perpendicular to the beam axis. This photon flux is calculated by taking into account the mechanical parameters such as the rotation of the gantry and collimator, the position of the main collimators, the used accessories (such as shielding blocks or wedges), the positions of all MLC leafs and the fractional beam-on time (i.e. the number of delivered beam pulses calibrated in Monitor Units (MU)). For this purpose, the absorption characteristics of the different mechanical parts are characterised in terms of changes in photon intensity and photon energy spectrum, on the beam axis as well as off-axis.

Subsequently, the photon flux is transformed into a volumetric photon beam by tracing each divergent ray and scaling the photon intensity according to the inverse square law. For this an appropriate algorithm can be used. The calculations can be done in a carthesian coordinate system or along a divergent grid and are preferably subsequently transformed to a carthesian coordinate system. The final superposition is generally done onto a carthesian coordinate system.

For calculating the three-dimensional in-air photon distribution based on the measured radiation beams generated by the radiotherapy apparatus, the so-called portal dose is measured by means of the portal imager as a function of gantry angle.

To be able to do so, a number of technical and analytical aspects need to be addressed:
- the gantry angle needs to be measured alongside the portal dose image acquisition;
- an accurate characterisation and calibration of the portal imager is mandatory. A calibration procedure for the portal imager is such that it takes into account the different pixel sensitivities of the portal imager as well as their spectral dependence and the impact of the mechanical construction of the portal imager on the measured image.
- a dedicated algorithm reconstructs the three dimensional photon cloud from the measured data. This algorithm takes into account the position of the imager at the time of acquisition and the response function of the portal imaging device.

The reconstruction of the three dimensional photon-cloud from the portal imager measurements involves numerous steps as is explained below.

Firstly, an image acquisition mode must be programmed on the portal imager, which is a megavolt portal imager, to allow individual image frame acquisitions per gantry angle whereby simultaneously the corresponding gantry angle is stored.

Secondly, as represented schematically in figure 3, a set of image transformation matrices needs to be determined to allow the conversion of the raw image acquired by the portal imager into a photon flux:
- Acquiring and storing an image without irradiation provides a correction matrix for background subtraction
- A relative sensitivity correction matrix corrects for the differences in sensitivity between the individual pixels of the portal imager. The imager is irradiated in its central position whereby the centre of the plane of the portal imager is, preferably, situated in the isocentre of the treatment apparatus, subsequently this centre of the imager is shifted over a limited distance along its plane in a first direction, i.e. in the longitudinal direction, and irradiated with the same field. The same procedure is repeated with a shift along its plane in a second direction, i.e. the lateral direction, perpendicular to the first direction. Edge detection on all images quantifies the mechanical shift. Normalising to the sensitivity of the central pixel, the relative difference between the central image and the shifted images allows calculation of the pixel sensitivity for all pixels.
- A matrix for the correction of scatter effects of the detector device construction, wherein scatter and backscatter effects are quantified by means of a series of image acquisitions for different field sizes of the radiation beam and different positions of the detector device. These corrections for the scatter effects of the mechanical construction of the portal imager, especially of the metallic construction parts of the support arm, are based on a carefully monitored position of the plane of the portal imager relative to these mechanical parts.
- A correction matrix for the spectral dependence of the portal imager. Because of the strong spectral dependence of some portal imaging devices, a correction for this is mandatory. The spectral dependence is normalized to the pixel sensitivity on the beam axis for a fixed field size for each nominal beam energy. Two important sources on spectral variations in the beam exist.
   ■ Firstly, the beam flattening filter in the accelerator head causes a beam hardening towards the centre of the beam by filtering out more low energy photons. These off-axis changes in beam spectrum can be modelled using Monte Carlo - based calculation techniques. A correction matrix can therefore be generated and applied to the imager plane, taking its position relative to the beam axis into account.
   ■ Secondly, all additional absorbers that are inserted into the treatment beam, such as shielding blocks, wedges and MLCs, give rise to an increase in scattered particles and have a beam hardening effect on the spectrum. The transmission spectrum is characterised individually for each absorber, as is the relative impact on the pixel sensitivity. The section of the portal image that received such transmission irradiation is corrected accordingly.
- Each acquired image is processed with the corresponding energy-dependent point spread function (PSF) of the portal imager to obtain the impeding photon flux.

Thirdly, the three dimensional photon-cloud is reconstructed through superposition of the contributions from each gantry angle. The individual contributions are converted to volumetric photon beams by forward- and backprojection from the plane of measurement along all divergent rays, taking into account the inverse square law. They are subsequently summed to obtain the total three dimensional photon-cloud.

The method and the apparatus of the invention introduce a new concept by making use of a three dimensional photon cloud in air, especially by obtaining a three dimensional photon cloud for arc therapy from measurements with a portal imager in treatment or apparatus verification procedures. According to the state of the art, treatment planning verification procedures are based on a two dimensional photon fluence or on a (one-, two or three dimensional) dose in a patient or phantom. Hence, prior art methods use radiation dose calculations and measurements, not three dimensional photon density distribution.

For patient specific verification of a treatment plan and regular verification of the radiotherapy apparatus, in particular of the linear accelerator, measurement and calculation methods are very similar. The main difference lies in the fact that for the patient specific verification, a treatment plan is generated by the treatment planning system, whereas, for the routine apparatus quality control dedicated delivery sequences can be artificially programmed outside of the treatment planning system to focus on the different aspects of the apparatus performance.

The invention is of course not restricted to the embodiments of the method and the apparatus described above. It is clear that other variants would also be conceivable without departing from the scope of the present invention.

Accordingly, in certain instances, it is sufficient to compare and evaluate for each gantry angle individually the three-dimensional in-air photon distribution predicted on basis of the treatment plan and the calculated three-dimensional in-air photon distribution based on the measured radiation. However, areas or zones presenting an unexpected low or high photon density may only be detected after superposition of the photon distribution of all gantry angles.

Notwithstanding in the above description a portal imager is disclosed for generating an image of the beams generated by the treatment apparatus, it is clear that any detector device, preferably a two dimensional detector device, that is capable of generating an image of the radiation field of the radiation beams can be used in the method and the apparatus, according to the invention. Further, the portal imager or the detector device is not necessarily fixed to the gantry but can also be formed by an external device.

Although the method of the invention is developed for the specific purpose of verifying the performance of the radiotherapy treatment apparatus in case of the latest developments in dynamic arc therapy, it can equally well be applied to all radiotherapy treatments using MV photon beams, ranging from conventional static open beam delivery to static gantry IMRT treatment delivery.

## Claims

1. Method for the verification of a radiotherapy treatment apparatus having a radiation source mounted to a gantry for directing one or multiple radiation beams to a target from one or from multiple angles of beam incidence, wherein the beams can be altered or modulated as a function of gantry angle, position or time, and for the verification of treatment preparation software and control software, the method comprising the steps of
- obtaining a treatment plan for treatment of a target in a patient or in a phantom or for apparatus performance control, said treatment plan comprising defining said radiation beams to be directed from one or from multiple angles of incidence;
whereby the method is **characterised in that** it comprises the steps of
- predicting a three-dimensional photon distribution in air on basis of said treatment plan;
- generating said radiation beams in air, wherein the radiation beams are defined by the treatment plan, while measuring radiation from said radiation beams by means of a detector device and calculating a corresponding three-dimensional in-air photon distribution taking into account the measured radiation and its angular distribution;
- comparing and evaluating the three-dimensional in-air photon distribution predicted on basis of said treatment plan and the calculated three-dimensional in-air photon distribution based on the measured radiation.

2. Method according to claim 1, wherein radiation from said radiation beams is measured as a function of gantry angle when rotating the gantry around a centre or an axis of rotation.

3. Method according to claim 1 or 2, wherein said three-dimensional photon distribution is predicted on basis of said treatment plan by superposing individual three-dimensional photon distributions of said radiation beams calculated for each gantry angle or by integrating the three dimensional photon distribution of said radiation beams over all angles.

4. Method according to any of claims 1 to 3, wherein said three-dimensional photon distribution is predicted on basis of said treatment plan by calculating the photon flux in a plane perpendicular to the central axis of said radiation beams, wherein this plane preferably comprises the isocentre through which the central axis of the radiation beams passes for different gantry angles, and subsequently transforming this photon flux into said three-dimensional photon distribution.

5. Method according to claim 4, wherein said photon flux is calculated taking into account mechanical parameters of the radiotherapy treatment apparatus such as absorption characteristics of mechanical parts of the apparatus, wherein these characteristics are expressed in terms of changes in photon intensity and/or photon energy spectrum on the central axis of the radiation beam as well as off this axis.

6. Method according to claim 4 or 5, wherein said photon flux is transformed into said three-dimensional in-air photon distribution by tracing each divergent ray of said radiation beams and scaling the photon intensity, preferably, according to the inverse square law.

7. Method according to any of claims 1 to 6, wherein measuring radiation from said radiation beams is performed by providing a portal imager defining a plane of measurement, preferably mounted to said gantry, whereby portal dose images generated by the portal imager are determined as a function of the gantry angle.

8. Method according to any of claims 1 to 7, wherein measuring radiation from said radiation beams comprises acquiring individual images of the radiation beams in a plane of measurement perpendicular to the central axis of this beam, whereby the images are stored together with the corresponding gantry angle.

9. Method according to claim 8, wherein a set of transformation matrices is determined for converting said images into a photon flux, these transformation matrices comprising at least one of the following:
- a correction matrix for background subtraction obtained by acquiring an image without generating a radiation beam;
- a relative sensitivity correction matrix for correcting differences in sensitivity between individual pixels of said detector device;
- a matrix for the correction of scatter effects of the detector device construction, wherein scatter and backscatter effects are quantified by means of a series of image acquisitions for different field sizes of the radiation beam and different positions of the detector device;
- a correction matrix for the spectral dependence of the detector device.

10. Method according to any of claims 7 to 9, wherein said measured images of the radiation beams are processed with said transformation matrices and with an energy-specific Point Spread Function of the detector device to obtain the impeding photon flux for the associated gantry angles.

11. Method according to claim 10, wherein said photon flux is transformed into an individual three-dimensional photon distribution for each gantry angle by forward and backward projection from said plane of measurement along all divergent rays of the radiation beam.

12. Method according to claim 11, wherein the three-dimensional photon distribution is determined by superposing the individual three-dimensional photon distributions for all gantry angles derived from the images of the radiation beam in said plane of measurement.

13. Radiotherapy treatment apparatus permitting comparing and evaluating a three-dimensional photon distribution predicted on basis of a treatment plan and a calculated three-dimensional photon distribution based on a measured radiation of a radiation beam, wherein this apparatus comprises
- a radiation source for generating a radiation beam, wherein the radiation source is mounted to a gantry that can be rotated around a centre or axis of rotation
- a portal imaging device that is capable of generating a portal image in response to said radiation beams;
- means for acquiring a gantry angle associated with a corresponding portal image and for storing this gantry angle alongside the corresponding portal image.
